**Europäisches Patentamt**

**European Patent Office**

**Office européen des brevets**

(19)

(11) Publication number: **0 148 290**
A1

# (12) EUROPEAN PATENT APPLICATION

(21) Application number: **83850331.6**

(22) Date of filing: **14.12.83**

(51) Int. Cl.⁴: **G 01 N 35/00**, G 01 N 1/34, C 12 Q 1/06

(43) Date of publication of application: **17.07.85 Bulletin 85/29**

(84) Designated Contracting States: **AT CH DE FR GB IT LI NL**

(71) Applicant: **FÖRSVARETS FORSKNINGSANSTALT, Box 27322, S-102 54 Stockholm (SE)**

(72) Inventor: **Linfors, Gunilla, Segelbatsvägen 6, S-902 91 Täftea (SE)**
Inventor: **Hallin, Pia, Korpralsvägen 48 C, S-902 53 Umea (SE)**

(74) Representative: **Robinson, Marianne et al, Försvarets civilförvaltning Patent Section Box 80012 Östermalmsgatan 87, S-104 50 Stockholm (SE)**

(54) **Method and device at the analysis of liquid samples.**

(57) The invention relates to a method and a device used in a preparatory step in the analysis of a liquid sample, for instance at the determination of its content of microorganisms. The invention makes possible a continuous, automatic concentration on a filter (3) of the substance to be determined before the analysis thereof, the filter being in the form of an automatically movable filter tape which is controlled by an operation unit.

EP 0 148 290 A1

0148290

Method and device at the analysis of liquid samples

The present invention relates to a method used as a preparatory step in the qualitative and quantitative analysis of liquid samples and is particularly valuable, when the substance to be determined is present in a low concentration. The invention also relates to a device for carrying out the method. By using the method and the device according to the invention even small amounts of microorganisms can be determined in liquid samples. The invention is also suitable for the determination of airborne microorganisms being collected in an appropriate liquid, usually water, before the analysis thereof.

In the analysis of a liquid sample, and particularly a water sample, as regards to its content of microorganisms it takes a long time before a reply can be obtained if the microorganisms must be cultivated. Therefore it is desirable to obtain a method of analysis that will give a more rapid reply. A big problem is that often there are too low concentrations of microorganisms for the use of for instance a biochemical method for the determination of the amount of microorganisms. Here an important step is to concentrate the microorganisms of the sample to be analyzed. There are methods available to concentrate microorganisms on filters but these methods are not automatic.

By means of the method and the device of the invention a continuous automatic concentration is obtained of the substance to be determined in the liquid sample, making it possible to carry out the analysis with a great accuracy even when using simple methods of analysis. The characteristics of the invention are evident from the subsequent patent claims.

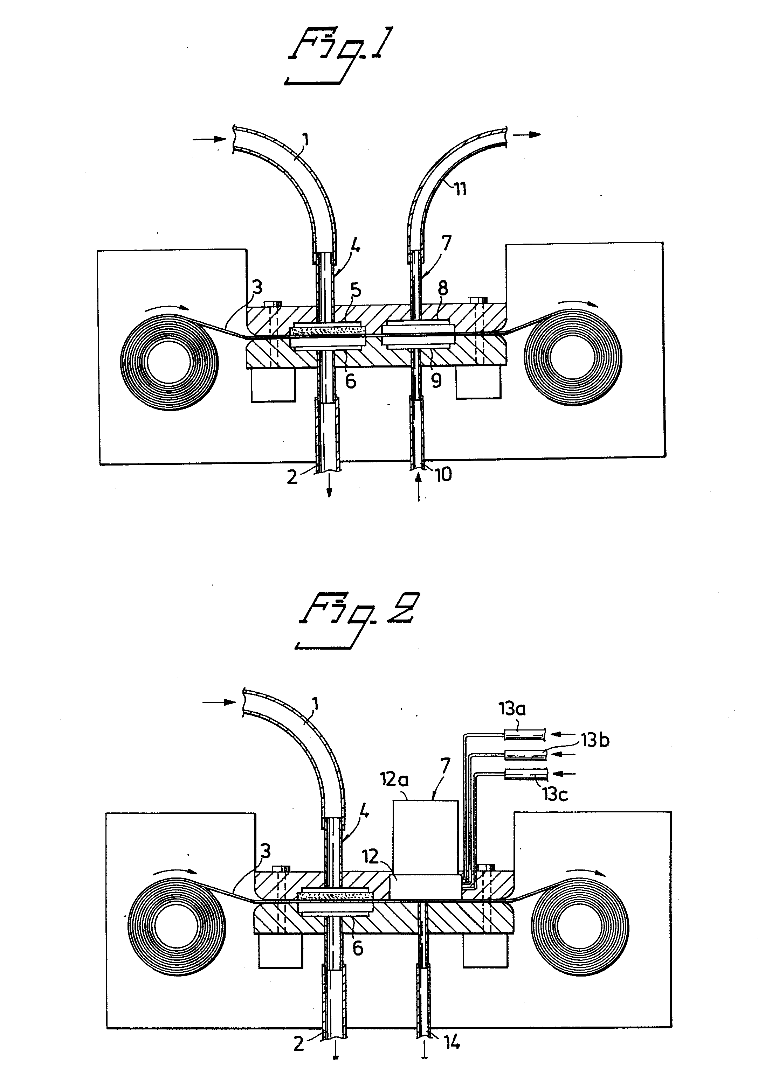

The invention will now be described in more detail to an illustrative but not delimiting purpose with reference to the attached drawings, where Figs. 1-3 show three embodiments of the device according to the invention for an automatic concentration of, for instance, microorganisms in a liquid sample before the analysis thereof.

The device of Fig. 1 comprises a filter 3 in the form of a movable filter tape that is brought to pass through two filter holders 4, 7. Each filter holder consists of an upper part 5, 8 and a lower part 6, 9. The upper parts of the filter holders are arranged in a common lid situated above the filter while the lower parts of the filter holders are arranged in a common seat situated below the filter. The liquid sample that is to be analyzed, is supplied by means of a peristaltic pump to the first filter holder 4 through an intake 1 connected to the upper part 5. The filter is permeable to the liquid vehicle of the sample causing the sample content of the substance that is to be determined, for instance microorganisms, to become concentrated on the filter while the filtrate is caused to pass through the lower part 6 of the filter holder through an outlet 2 into a waste.

After concentration has been carried out, the lid with the upper part 5 is lifted up and the filter with microorganisms is automatically moved forwards to the second filter holder 7. The automatic movement forwards of the filter is achieved by means of an operation unit. The lid is closed again, and a liquid being suitable for the elution of the microorganisms is pumped in from preferably the underneath side of the filter through the inlet 10. The microorganisms that have been concentrated on the upper side of the filter are thus eluted into the liquid and are removed from the device through the outlet 11 for a rapid determination thereof, for instance by means of a chemiluminescence analysis in a coventional manner.

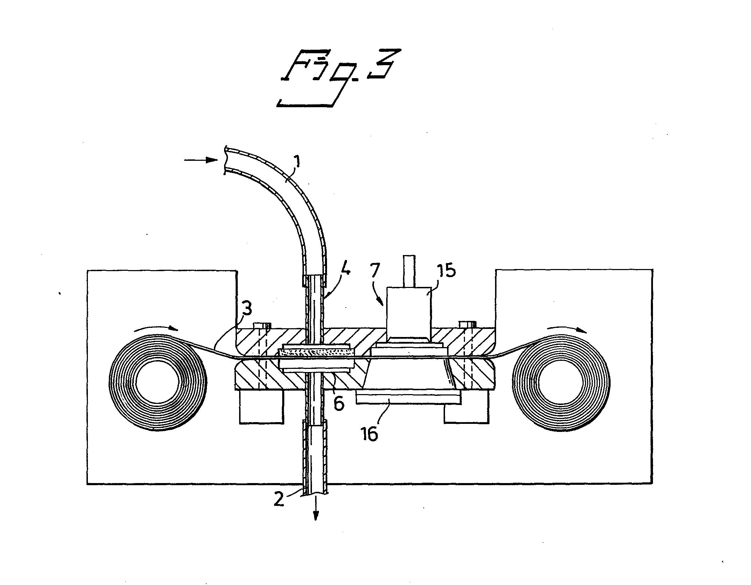

In the device of Fig. 2 the second filter holder 7 is in the form of a measuring unit. It is here possible to carry out a direct analysis of the concentrated sample by means of for instance chemiluminescence. Reagents for luminescence analysis are being injected into the measuring unit 12 through an inlet 13, for instance luminol 13a, perborate 13b and sodium hydroxide 13c. The emitted light is observed by means of a detector, for instance a photomultiplier tube 12a. With certain predetermined analysis conditions the emitted light is a measure of the amount of microorganisms.

In the device of Fig. 3 the second filter holder 7 is in the form of a punch.  Said punch 15 cuts out that part of the filter, on which the sample has been concentrated, and so the filter falls down into a container 16, in which container the analysis can be carried out in a conventional manner.  The container 16 may be a petri dish with a nutrient medium for microorganisms.  It is then possible to carry out conventional  microbiological cultivation, for instance in accordance with the standards that are established for water samples.

In the description above the concentration is carried out semicontinuously.  The invention also makes it possible to carry out the concentration and the analysis altogether continuously.

0148290

What is claimed is:

1. A method at the qualitative and quantitative analysis of liquid samples comprising in a liquid vehicle small amounts of a substance to be determined, for instance microorganisms, characterized in that the sample is taken up on a filter in the form of a movable filter tape, which filter is permeable to the liquid vehicle of the sample but not permeable to the substance to be determined in the sample, the filter tape being automatically moved from a position, where the sample is being concentrated on the filter as regards the substance to be determined, to a position where the sample is being analyzed, either directly on the filter or in a subsequent step after elution of the sample from the filter.

2. Method according to claim 1, characterized in that a chemiluminescence reaction is utilized in the analysis of a liquid sample, particularly a water sample, as regards its content of microorganisms after the concentration thereof on the filter, in which reaction for instance luminol and perborate are added at an alkaline pH and the emitted luminescence is observed by means of a detector.

3. A device for carrying out the method according to claim 1 or 2 for an automatic concentration of liquid samples as regards the substance to be determined in the sample, characterized in that it comprises an inlet (1) and an outlet (2) for the liquid sample, a pump for the supply of the liquid sample, a filter (3) in the form of a movable filter tape, the filter being permeable to the liquid vehicle of the sample but not to the substance to be determined in the sample, an operation unit for the control of the filter tape movement through the device, a first filter holder (4), the upper part (5) thereof being connected to the inlet (1) and the lower part (6) thereof being connected to the outlet (2), and a second filter holder (7) also comprising an upper part (8) and a lower part (9), the filter in its movement through the device from the first filter holder to the second filter holder being arranged to pass between the upper parts and the lower parts of the two filter holders.

4. Device according to claim 3, characterized in that the lower part (9) of the second filter holder (7) is connected to an inlet (10) for the supply to the filter of a suitable liquid for the elution from the filter of the sample being concentrated in the first filter holder (4), and that the upper part (8) of the second filter holder is provided with an outlet (11) for the eluted sample, whereafter the sample is analyzed in a conventional manner.

5. Device according to claim 3, characterized in that the second filter holder (7) is a measuring unit (12) for a direct analysis of the sample being concentrated in the first filter holder (4), the measuring unit being provided with inlets (13) for the reagents that are required for the analysis and an outlet (14) for reagents used and for the sample eluted from the filter.

6. Device according to claim 5, characterized in that the measuring unit (12) is provided with inlets (13) for reagents suitable for luminescence tests for the determination of the microorganisms being present in the sample, for instance luminol (13a), perborate (13b) and sodium hydroxide (13c), and a photomultiplier tube (12a) for the observation of the luminescence emitted.

7. Device according to claim 3, characterized in that the upper part (8) of the second filter holder (7) is a punch (15) for the cutting out of samples of the filter tape and so of the sample being concentrated on the filter, and that the lower part (9) of the second filter holder is a container (16), in which container the analysis can be carried out in a conventional manner.

8. Device according to claim 7, characterized in that the container (16) is a petri dish with a nutrient medium for microorganisms, the sample content of microorganisms being determined by means of conventional microbiological cultivation.

9. Device according to any one of claims 3-8, characterized in that the upper parts (5, 8) of the first and second filter holders are arranged in a common lid and that the lower parts (6, 9) of the first and second filter holders are arranged in a common seat.

0148290

*Fig.1*

*Fig.2*

*Fig.3*

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| X | WO-A-8 304 309  (ISMATEC SA)  * Abstract;  page 7, claims 1,2; figure * | 1,3,7, 9 | G 01 N  35/00 G 01 N   1/34 C 12 Q   1/06 |
| X | US-A-4 283 490  (C.J. PLAKAS) * Column 2, lines 7-45; column 5, lines 44-65; figure 3 * | 1,3,5 | |
| A | | 6 | |
| X | GB-A-1 438 934  (NATIONAL RESEARCH DEVELOPMENT CO.) * Whole document; figure 1 * | 1,3,5, 9 | |
| A | CHEMICAL ABSTRACTS, vol. 70, no. 1, 6th January 1969, page 624, no. 6468a, Columbus, Ohio, US; W.S. OLENIACZ et al.: "Chemiluminescent method for detecting microorganisms in water" & ENVIRON. SCI. TECHNOL. 1968, 2(11), 1030-3 | 1,2,6 | **TECHNICAL FIELDS SEARCHED (Int. Cl. ³)**  G 01 N   1/00 G 01 N  35/00 G 01 N  33/00 C 12 Q   1/00 |
| A | US-A-3 690 837  (S. WITZ et al.)  * Abstract;  column 1, line 66 - column 2, line 12; claim 1 * | 1,2,4, 6 | |
| | ---          -/- | | |

The present search report has been drawn up for all claims

| Place of search THE HAGUE | Date of completion of the search 23-07-1984 | Examiner ERRANI C. |
|---|---|---|

European Patent
Office

**EUROPEAN SEARCH REPORT**

0148290

Application number

EP  83 85 0331

## DOCUMENTS CONSIDERED TO BE RELEVANT

Page  2

| Category | Citation of document with indication, where appropriate, of relevant passages | Relevant to claim | CLASSIFICATION OF THE APPLICATION (Int. Cl. 3) |
|---|---|---|---|
| A | US-A-4 176 007  (E.L. JEFFERS et al.)<br>* Abstract; claim 1 * | 2,6 | |
| A | BE-A-  547 621  (CHIRANA PRAHA)<br>* Page  1, line 1 - page 2, line 16 * | 8 | |

**TECHNICAL FIELDS SEARCHED (Int. Cl. 3)**

The present search report has been drawn up for all claims

| Place of search<br>THE HAGUE | Date of completion of the search<br>23-07-1984 | Examiner<br>ERRANI C. |
|---|---|---|